(19) **Europäisches Patentamt**
**European Patent Office**
**Office européen des brevets**

(11) **EP 3 968 018 B1**

(12) **EUROPEAN PATENT SPECIFICATION**

(45) Date of publication and mention
of the grant of the patent:
**30.10.2024 Bulletin 2024/44**

(51) International Patent Classification (IPC):
*G06F 18/23213* (2023.01)   *G06F 18/2433* (2023.01)
*G01N 29/02* (2006.01)   *G01N 29/036* (2006.01)
*G01N 29/44* (2006.01)   *G01N 33/00* (2006.01)
*G01N 21/552* (2014.01)

(21) Application number: **20306023.1**

(22) Date of filing: **14.09.2020**

(52) Cooperative Patent Classification (CPC):
**G06F 18/23213; G01N 29/022; G01N 29/036;**
**G01N 29/4436; G06F 18/2433;** G01N 21/553;
G01N 33/0034; G01N 2291/0255; G01N 2291/0256;
G06F 2218/12

(54) **SENSOR FOR IDENTIFYING A FLUID SAMPLE AND METHOD FOR APPLYING A QUALIFICATION TEST TO SUCH A SENSOR**

SENSOR ZUR IDENTIFIZIERUNG EINER FLUIDPROBE UND VERFAHREN ZUR ANWENDUNG EINES QUALIFIKATIONSTESTS AN SOLCH EINEM SENSOR

CAPTEUR D'IDENTIFICATION D'UN ÉCHANTILLON DE FLUIDE ET PROCÉDÉ D'APPLICATION D'UN TEST DE QUALIFICATION POUR UN TEL CAPTEUR

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**16.03.2022 Bulletin 2022/11**

(73) Proprietor: **Aryballe**
**38000 Grenoble (FR)**

(72) Inventors:
• **CARITU, Yanis**
**38000 Grenoble (FR)**
• **HARBINE, David**
**38000 Grenoble (FR)**

(74) Representative: **Bonnet, Michel**
**Cabinet Bonnet**
**93, rue Réaumur**
**75002 Paris (FR)**

(56) References cited:
EP-A1- 1 566 633   CN-A- 107 741 471
FR-A1- 3 091 346   US-A1- 2020 256 793

• JENDRIKE NINA ET AL: "ISO 15197: 2013 Evaluation of a Blood Glucose Monitoring System's Measurement Accuracy", JOURNAL OF DIABETES SCIENCE AND TECHNOLOGY DIABETES TECHNOLOGY SOCIETY REPRINTS AND PERMISSIONS, vol. 11, 1 January 2017 (2017-01-01), pages 1275 - 1276, XP055781073
• NATALE C D ET AL: "SENSOR ARRAYS CALIBRATION WITH ENHANCED NEURAL NETWORKS", SENSORS AND ACTUATORS B: CHEMICAL, ELSEVIER BV, NL, vol. B19, no. 1/03, 1 April 1994 (1994-04-01), pages 654 - 657, XP000449924, ISSN: 0925-4005, DOI: 10.1016/0925-4005(93)01115-K

## Description

### BACKGROUND OF THE INVENTION

The invention relates to a sensor configured to input a fluid sample and to output a corresponding signature obtained from an electric signal characterizing the fluid sample. It also relates to a method for administering a qualification test to such a sensor and to a method for manufacturing a certified sensor.

More specifically, it relates to a sensor comprising:

- at least one reactive site; and

- at least one transducer configured to measure at least one physical property change induced by an interaction of the fluid sample with said at least one reactive site and to output said electric signal.

It must be noted that the signature obtained from the above-mentioned electric signal may be:

- the electric signal itself when no specific processing is performed on it before outputting it; or

- the result of a specific processing on the signal provided at the output of the transducer, for example including an amplification, a sampling, a normalization, a feature extraction, a (statistical) data analysis or else.

### DESCRIPTION OF THE PRIOR ART

Such a sensor, in case of an electronic nose, is for example used for detecting, discriminating and identifying volatile organic compounds in the fluid sample. It can be used in various industrial fields such as:

- the fragrance industry, for example to measure, identify and compare, for studying or designing pure or mixtures of olfactory compounds,
- the protection of environment, in particular for olfactory pollution or quality of more or less confined environments monitoring,
- the surveillance of manufacturing sites, likely to be contaminated by volatile materials potentially dangerous or odorous,
- health, for example to offer a substitute for smell to people suffering from anosmia or to detect volatile biological markers,
- the food industry, for example for detecting contaminations in a food manufacturing and/or distribution chain,
- any industrial field where the control of any odorous product may be useful.

An example is known from patent application WO 2019/053366 A1. This example, called NeOse Pro (registered trademark) has been marketed by Aryballe Technologies company since 2018. It comprises a processor that is capable of outputting a N-component digital signature from each electric signal provided by the transducer, wherein said signature is specific to each detected odor. In general, integer N is less or equal to the number of reactive sites. By learning on various fluid samples and under various conditions, this sensor is potentially able to identify all odors.

But detecting, discriminating and identifying odors is generally considered highly subjective. As a consequence, qualifying such a sensor is difficult and usually considered biased. It is also difficult to compare different sensors, wherein some of them may have reactive sites and transducers made of different technologies. In particular, biosensors of reactive sites are generally deposited or grafted on a substrate at nanoscale and it is difficult to assess accurately the quality of such a deposition or grafting without improved and accurate qualifying methods.

### SUMMARY OF THE INVENTION

It may thus be desirable to design a sensor capable of overcoming at least some of the above-mentioned problems and constraints.

The invention is defined by a method of administering a qualification test to a sensor according to claim 1 and a method of manufacturing a certified sensor according to claim 12.

Thus, thanks to a metrics based on a clustering quality score of several signatures obtained from several reference fluid samples, the sensor can be certified to have passed a qualification test which makes it possible to impartially technically compare it to others or guarantee a certain objective level of quality.

Optionally, the metrics further comprises one or more of a signal-to-noise ratio, a limit of detection, an index of repeatability and an index of reproducibility.

Optionally also, a sensor according to the invention may further be configured to input the signature of the fluid sample to a classifier connected to a database of reference signatures of respective reference fluid samples in order to have the signature of the fluid sample associated to one of said reference signatures, wherein the metrics further comprises an index of classification performance.

Optionally also, the index of classification performance is based on a confusion matrix of the reference signatures.

Optionally also, the plurality of reference fluid samples each comprise a volatile organic compound.

Optionally also, a sensor according to the invention may further comprise a plurality of reactive sites and each of said reactive sites comprises a chemical component that has adsorbing properties for a volatile organic compound, such as a peptide immobilized on a substrate, or a polymer coated on a surface.

Optionally also, the at least one transducer comprises:

- a surface plasmon resonance imaging system configured to measure a change in a refractive index thanks to a plasmonic effect; or
- a Mach-Zehnder interferometer configured to measure a phase shift due to a change in a refractive index; or
- a nano- or micro-electromechanical system configured to measure a change in resonance frequency of a vibrating membrane thereof.

Optionally, computing the metrics comprises:

a) selecting a plurality of reference analytes,
b) conditioning them in the plurality of reference fluid samples, with the plurality of reference analytes in concentrations exceeding a predefined level,
c) outputting the corresponding signatures of the plurality of reference fluid samples over a plurality of measurement cycles,
d) annotating the corresponding signatures of the plurality of reference fluid samples with their true reference analyte label for clustering,
e) computing, for each reference fluid sample $S_i$, a combination $a_i$ of intra-cluster distances for the corresponding signature within the cluster thereof,
f) computing, for each reference fluid sample $S_i$, at least one combination of extra-cluster distances $b_{i,j}$ for the corresponding signature with the signatures in the cluster(s) different from the cluster thereof,
g) computing a validity index combining the intra-cluster distances and the extra-cluster distances for each of some or all of the reference fluid samples.

Optionally, the clustering quality score is based on the one or more validity index(es) computed at step g).
Optionally also, a method according to the invention may further comprise repeating at least steps a) through c), for example steps a) to d), for a plurality of measurement runs for a same sensor, each run being distant in time of about a predefined interval, wherein the metrics further comprises an index of repeatability computed as a composite of at least one of:

- a clustering quality score computed over the plurality of measurement runs;

- an index of dispersion of intensities of the signatures of the fluid samples for the plurality of measurement runs; and

- an index of dispersion of the signatures normalized by their respective intensities of the fluid samples for the plurality of measurement runs.

Optionally also, a method according to the invention may further comprise repeating at least steps a) through c), for example steps a) to d), for a plurality of sensors, wherein the metrics further comprises an index of reproducibility computed as a composite of at least one of:

- a clustering quality score computed over the plurality of sensors;

- an index of dispersion of intensities of the signatures of the fluid samples for the plurality of sensors; and

- an index of dispersion of the signatures normalized by their respective intensities of the fluid samples for the plurality of sensors.

Optionally also, a method according to the invention may further comprise repeating at least steps a) through c), for example steps a) to d), for a plurality of measurement runs, each run being distant in time of about a predefined interval, and, for each measurement run, repeating steps a) through c), for example steps a) to d), for a plurality of sensors, wherein the metrics further comprises an index of repeatability and reproducibility computed as a composite of at least one of:

- a clustering quality score computed over the plurality of measurement runs and the plurality of sensors;

- an index of dispersion of intensities of the signatures of the fluid samples for the plurality of measurement runs and the plurality of sensors; and

- an index of dispersion of the signatures normalized by their respective intensities of the fluid samples for the plurality of measurement runs and the plurality of sensors.

A method for manufacturing a certified sensor is further proposed, comprising:

- providing a sensor configured to input a fluid sample and to output a corresponding signature obtained from an electric signal characterizing the fluid sample, the sensor comprising at least one reactive site and at least one transducer configured to measure at least one physical property change induced by an interaction of the fluid sample with said at least one reactive site and to output said electric signal;

- applying the aforementioned method for administering a qualification test to the sensor;

- checking that the metrics computed by administering the qualification test matches at least one predefined qualifying criterion; and

- certifying the sensor as far as said metrics matches said at least one predefined qualifying criterion.

BRIEF DESCRIPTION OF THE DRAWINGS

The invention will be better understood with reference to the following description, given purely as an example, with reference to the appended drawings in which:

- Fig. 1 diagrammatically represents the general structure of a sensor configured to input a fluid sample and to output a corresponding signature, according to an embodiment of the invention,

- Fig. 2 shows a typical example of an image produced by a transducer of the sensor in Fig. 1, when said transducer comprises a surface plasmon resonance imaging system or a Mach-Zehnder interferometer, whereon reactive sites of the sensor are visible,

- Fig. 3 illustrates superimposed time diagrams of response signals, or sensorgrams, for example obtained from an image such as the one of Fig. 2,

- Fig. 4 illustrates an olfactory signature obtained from a processing of the reflectance signals in Fig. 3,

- Fig. 5 illustrates the successive steps of a method for using the sensor in figure 1 and obtaining an olfactory signature of a fluid sample,

- Fig. 6 illustrates the successive steps of a method for administering a qualification test to the sensor in figure 1, and

- Fig. 7 illustrates the successive steps of a method for manufacturing the sensor in figure 1.

DESCRIPTION OF PREFERRED EMBODIMENTS

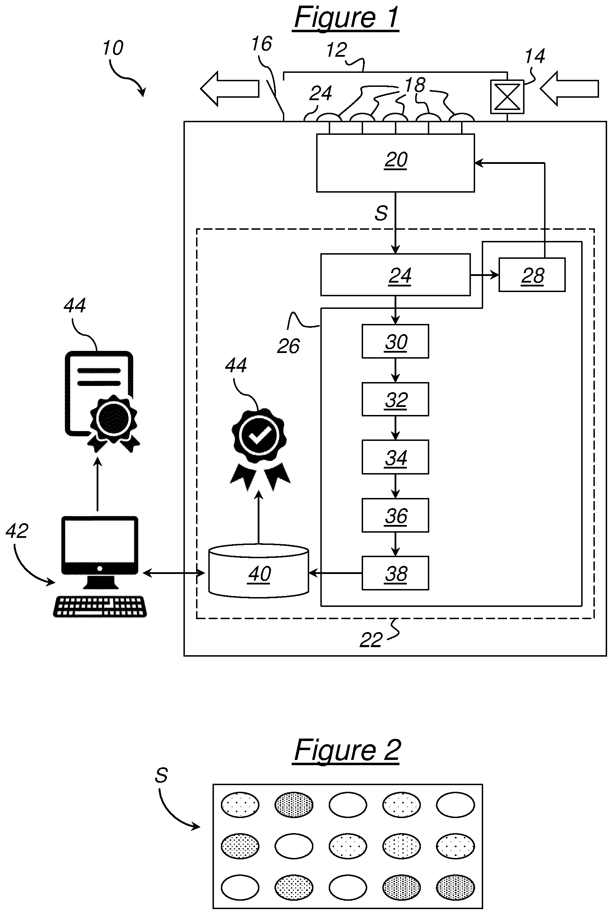

The electronic sensor device 10 shown schematically in Figure 1 is a non-limiting example of a sensor according to the invention for a non-limiting application, i.e. odor identification. It includes a chamber 12 designed to input a fluid sample, for example a gaseous sample. To do this, it may have a suction device 14 designed to draw the fluid sample from outside chamber 12 and bring it inside. It also may have an outlet 16 that can be selectively closed to keep the fluid sample in chamber 12 or opened to allow the fluid sample to be removed from chamber 12 and replaced by another fluid sample by activating the suction device 14. Both suction device 14 and outlet 16 are optional and could easily be deleted in an electronic sensor device according to the invention.

In its chamber 12, sensor device 10 includes several olfactory sensors on respective reactive sites 18, for example about sixty, designed to interact with volatile organic compounds likely to be present in the fluid sample kept in chamber 12, by emission of these compounds. Each olfactory sensor is, for example, a biosensor designed to interact with a particular family of volatile organic compounds. In practice, each olfactory sensor on each reactive site 18 may include a molecule, such as a peptide immobilized on a substrate or a polymer coated on a surface, complementary to the volatile organic compounds of the family associated with that olfactory sensor. In the example described, the reactive sites 18 are arranged in a matrix on a positioning grid, i.e. they are respectively located at the centers of the cells of this grid.

The reactive sites 18 are associated with at least one transducer 20, said transducer 20 arranged and configured to measure at least one physical property change induced by an interaction of the fluid sample with said reactive sites 18. The transducer 20 outputs an electric signal S that characterizes the fluid sample since it is representative of volatile organic compounds with which the olfactory sensors can interact in chamber 12.

More precisely, the transducer 20 may be a Surface Plasmon Resonance (SPR) imaging system configured to measure any change in a refractive index due to an interaction of the fluid sample with any reactive site thanks to a plasmonic effect. Such a transducer includes a light emitter, an optical prism and a camera for outputting an electric signal S which is a grayscale image sequence of the reactive sites 18.

Figure 2 shows a schematic example of a typical grey scale image produced by such a SPR imaging system, on which the reactive sites 18 of sensor device 10 are visible with their luminance values characterizing the volatile organic compounds with which they may have interacted. Note that in the example described, the reactive sites 18 are circular. But due to a tilt of the camera with respect to the positioning grid of the reactive sites 18, the areas they occupy in the greyscale image sequence S are ellipses.

In a variant, the transducer 20 may be a Mach-Zehnder interferometer system configured to measure any change in a refractive index due to an interaction of the fluid sample with any reactive site thanks to a detectable phase shift between a reference arm of the interferometer and a sensing arm whereon any reactive site is disposed. Such a transducer outputs an electric signal S which is a phase shift image sequence of the reactive sites 18.

In another variant, the transducer 20 may be a nano- or micro-electromechanical system (NEMS or MEMS) configured to measure a change in resonance frequency of a vibrating membrane whereon a reactive site is arranged. The reactive sites 18 are for example arranged on a matrix of NEMS or MEMS vibrating membranes in order to output an electric signal S which is a resonance frequency shift signal sequence of the reactive sites 18.

Whatever the transducer 20, the general idea remains to functionalize reactive sites using olfactory sensors (i.e. biosensors, polymers, carbon nanotubes, etc.) in such a way that they adsorb and desorb volatile organic compounds in a differentiated way, to get a differentiated molecular interaction response from the reactive sites, and to amplify the response in the form of an electrical signal S using a physical transduction device.

In the particular case sensor device 10 is just made of the chamber 12 with the reactive sites 18 and the transducer 20, then the electrical signal S at output constitutes a signature of the fluid sample in the chamber 12 whatever it is on an analog or digital form.

But back to Figure 1, sensor device 10 generally further includes several functional modules that will be described below. In the example described, these functional modules are software. For example, sensor device 10 includes a computer 22 with a central processing unit 24 and an associated memory area 26 wherein several computer programs or several functions of the same computer program are stored. These computer programs contain instructions to be executed by the central processing unit 24 for performing the functions of the software modules. They are presented as distinct, but this distinction is purely functional. They could also be grouped together according to any possible combination into one or more computer programs. Their functions could also be at least partly micro-programmed or micro-wired in dedicated integrated circuits, such as digital circuits. Thus, as a variant, the computer 22 could be replaced by an electronic device only made of digital circuits (without a computer program) for performing the same actions. The abovementioned functions could also be distributed remotely, for example in a cloud computing environment, such as in a perspective of crowdsensing.

Sensor device 10 thus first of all includes a software module 28, to be executed by the processing unit 24, for controlling the suction device 14 (when applicable), the air outlet 16 (when applicable too) and the transducer 20.

It further includes an optional but advantageous software module 30, to be executed by the processing unit 24, for selecting, among the reactive sites 18, a subset of sites sensitive to a selection of volatile organic compounds. These volatile organic compounds may vary from one fluid sample to another so that the selection of reactive sites 18 made by software module 30 can also vary and be parameterized. The selected subset contains for example $N \geq 1$ reactive site(s), or advantageously several reactive sites ($N \geq 2$).

When the transducer 20 is a SPR imaging system, sensor device 10 further includes a software module 32, to be executed by the processing unit 24, to extract N reflectance signals, respectively representative of the interactions of the N selected reactive sites 18 with the volatile organic compounds concerned, from the luminance values specific to these N selected reactive sites 18 in an image sequence S provided by the camera of the SPR imaging system. These reflectance signals are for example expressed as a percentage according to a ratio of luminance values obtained with a transverse polarized light on luminance values obtained with the same light polarized at 90 degrees for each of the N selected reactive sites 18.

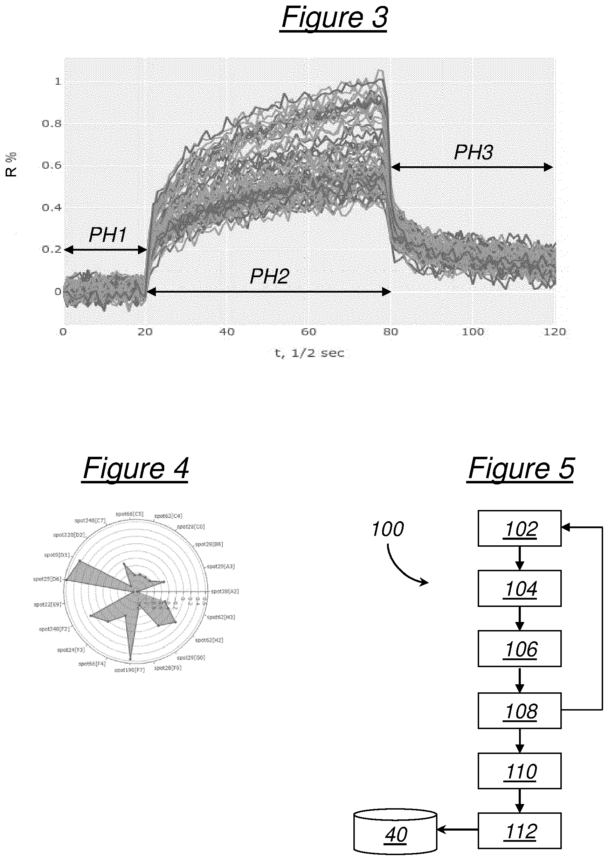

Figure 3 illustrates superimposed time diagrams of N = 19 reflectance signals obtained on a fluid sample according to a predefined measurement protocol wherein:

- the reactive sites 18 are exposed to a dry air environment without fluid sample during a first reference phase PH1,

- they are then exposed to the fluid sample during a second analytical phase PH2 of adsorption, and

- they are finally exposed again to the dry air environment without fluid sample during a third final phase PH3 of desorption.

A dry environment is defined as ambient air with a low mass fraction of water vapor, i.e. less than 500 ppm (parts-per-million) or even less than 100 ppm, and preferably less than 10 ppm, which is equivalent to a relative humidity of less than 0.1% at 4°C. Such dry air can for example be obtained by using silica gel or by extracting air from a frozen environment.

Sensor device 10 further includes a software module 34, to be executed by the processing unit 24, for selecting a time window for analysis of the N reflectance signals in order to extract N components of an olfactory signature representative of the fluid sample under study.

Sensor device 10 further includes a software module 36, to be executed by the processing unit 24, for obtaining the N components of the aforementioned olfactory signature from the N reflectance signals. This obtaining may include a correction of the N reflectance signals extracted in the selected time window. This correction will be detailed later with reference to figure 5. It consists mainly of two components: a correction of a drift of the olfactory sensors in the reactive sites 18 which is well known and will not be detailed, and a correction by subtraction from a reference value which depends on the predefined measurement protocol. It makes it possible to obtain N corrected reflectance signals. Each of the N components of the olfactory signature results for example directly or indirectly from the calculation of a statistical value representative of a respective one of the N corrected reflectance signals in the selected time window. It can simply be a scalar mean value in this time window. It can also be a more complex scalar or vector statistical value.

An example of a N = 19 components olfactory signature represented in a pie chart is shown in Figure 4, for example obtained from a sequential execution of software modules 28 to 36, i.e. based on reflectance signals such as the ones of Fig. 3.

In particular, the sequential execution can be repeated several times to obtain several successive olfactory signatures of the same fluid sample that can then be statistically processed, for example by averaging or otherwise, to obtain an improved olfactory signature. For this purpose, sensor device 10 optionally but advantageously includes a software module 38, intended to be executed by the processing unit 24, for the statistical processing of several N-component olfactory signatures. In particular, this can be a simple averaging of the olfactory signatures' components to obtain a centroid olfactory signature.

When the transducer 20 is a Mach-Zehnder interferometer system, the software module 32 is adapted in a way known *per se* to extract N phase shift signals instead of the N reflectance signals. These phase shift signals are for example expressed in Radian. The software modules 34 and 36 are also simply adapted to the phase shift signals to select the appropriate time window and obtain the olfactory signature.

When the transducer 20 is a NEMS or MEMS, the software module 32 is adapted in a way known *per se* to extract N frequency shift signals instead of the N reflectance signals. These reference shift signals are for example expressed in Hz. The software modules 34 and 36 are also simply adapted to the reference shift signals to select the appropriate time window and obtain the olfactory signature.

It should also be noted that each olfactory signature obtained from the electrical signal S by software processing may be:

- a raw signature, i.e. a vector the N components of which are representative of the response intensities of the N selected reactive sites to the fluid sample,

- a normalized signature, i.e. a vector the N components of which are those of the raw signature but normalized using for instance the L2 normalization,

- a simplified signature obtained from a principal component analysis of the raw or normalized signature, i.e. a simplified vector the $n \leq N$ components of which are the projections of the raw or normalized signature in the orthonormal reference system defined by the n first principal axes whereon the projections exceed a predefined threshold.

More generally, the simplified signature results from a dimension reduction. The principal component analysis may thus be replaced by a singular value decomposition, a multidimensional scaling, or any other appropriate dimension reduction algorithm.

When the olfactory signature results from a processing that includes a normalization, whatever it is a normalized signature or a simplified signature based on a normalized signature, the original intensity of the signature, i.e. its norm, can be kept as an additional information associated with the resulting olfactory signature.

Sensor device 10 further includes a memory zone 40 for storing each obtained olfactory signature.

The use of sensor device 10 for outputting an olfactory signature from a fluid sample will now be detailed with reference to Fig. 5.

At a first step 102 of a method 100 for outputting an olfactory signature from a fluid sample, such a method corresponding to a measurement cycle, sensor device 10 is arranged so that the olfactory sensors of its reactive sites 18 can be exposed to the fluid sample. The processing unit 24 thus executes the software module 28 to control the suction device 14, the air outlet 16 and the transducer 20. More precisely, this step may include the abovementioned first reference phase, second analytical adsorption phase and third final desorption phase. During these three exposure phases, the transducer 20 outputs an electric signal S that characterizes the fluid sample and transmits it to computer 22.

At following step 104, the electric signal S is received by computer 22 in the form of an image sequence when the transducer is a SPR imaging system. The processing unit 24 then runs software modules 30 and 32 to obtain N reflectance signals representative of the image sequence S for each of the N selected reactive sites. The three previously defined exposure phases can be seen very clearly in Fig. 3: the first reference phase PH1 extending from 0 to about 20 on the x-axis (expressed in half-seconds, i.e., for example, according to a time sampling at 2 Hz), the second analytical adsorption phase extending from about 20 to about 80, and the third final desorption phase starting at about 80.

At following step 106, the processing unit 24 runs the software module 34 for the selection of a time window for the analysis of the reflectance signals. In particular, it has been experimented that two time windows are particularly relevant for this type of three exposure phases. A first relevant time window covers the last part, for example the twenty last samples from 61 to 80, of the analytical adsorption phase PH2 in Fig. 3. A second relevant time window covers the first part, for example the twenty first samples from 81 to 100, of the final desorption phase PH3 in Fig. 3. One of these two time windows is therefore advantageously selected at this step. Alternatively, several time windows could be selected at this step to obtain a more complex olfactory signature with vector components, in particular a combination of the two abovementioned time windows. More generally, the whole available measurement time window could be taken into account by a machine learning system that would be able to extract other features.

At following step 108, the processing unit 24 runs the software module 36 to obtain an N-component olfactory signature. In particular, taking into account the aforementioned three-phase exposure of the olfactory sensors of reactive sites 18, the aforementioned correction by subtraction of a reference value can consist of subtracting the observed shift of each of the reflectance signals in the first reference phase PH1 from the respective values of these signals in the analytical phase PH2. For each reflectance signal, this shift is for example the average of the values of the signal in the reference phase PH1.

Steps 102 to 108 can be repeated as many times as desired, without changing the selections made in steps 104 and 106, to obtain multiple N-component olfactory signatures.

At a subsequent step 110 if steps 102 to 108 have been executed several times, processing unit 24 executes the software module 38 for statistical processing of the resulting olfactory signatures and obtaining a signature, e.g. averaged, which is then stored in memory 40 at a final step 112.

According to the invention, sensor device 10 is certified to have passed a qualification test comprising computing a metrics by checking that said metrics matches at least one predefined qualifying criterion, wherein the metrics comprises, for a plurality of reference fluid samples, a clustering quality score of respective signatures of the plurality of reference fluid samples.

To this end and back to Fig. 1, the method 100 is executed as a measurement cycle at least once for each of several reference fluid samples and several respective olfactory signatures are obtained that are stored in memory 40 and then sent to a computer 42, or directly sent to the computer 42. Each reference fluid sample for example includes an identified volatile organic compound to be detected by a corresponding reactive site and is *a priori* clustered in one of several predefined clusters so that the signatures obtained by execution of the measurement cycles 100 are also *a priori* clustered accordingly.

A method for administering a qualification test to sensor device 10 and certifying it is then executed by the computer 42. Administering the qualification test at least includes computing a metrics comprising a clustering quality score on the *a priori* clustered signatures. Certifying sensor device 10 at least includes checking that said clustering quality score matches a qualifying criterion, for example matches or exceeds a predefined threshold when said threshold is a floor, or matches or keeps under a predefined threshold when said threshold is a ceiling. The threshold, or more generally the qualifying criterion, may be defined based on a specification of an application of sensor device 10. When sensor device 10 has passed the qualification test, i.e. when the qualifying criterion has been matched, then a certificate 44 can be obtained and associated to sensor device 10 either by being stored therein, indicated thereon or included in a document associated thereto.

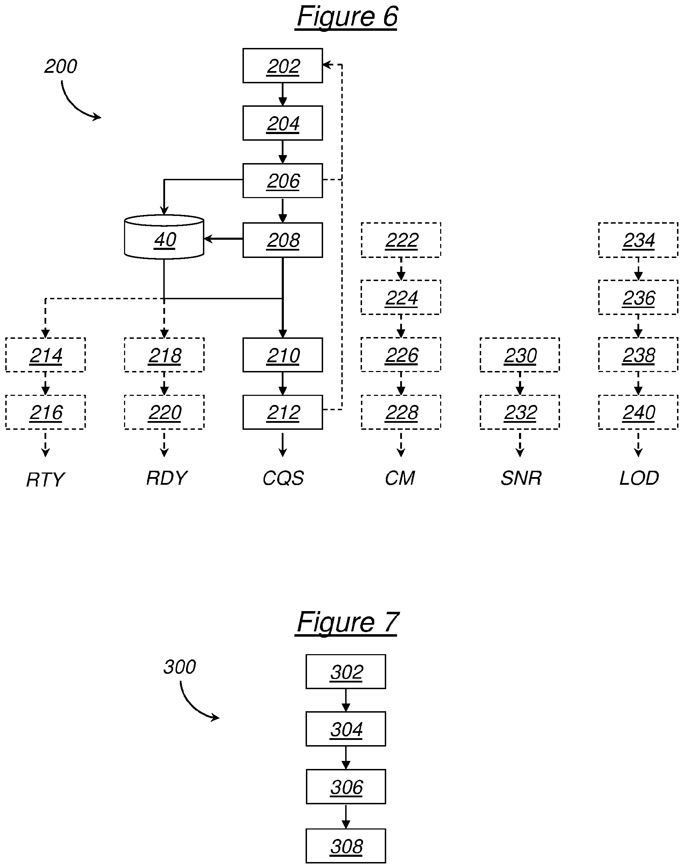

As illustrated in Fig. 6, a detailed method 200 for administering a qualification test to sensor device 10 may start at step 202 by selecting a plurality of reference analytes each of which defining a cluster. Each reference analyte is made of one or more molecules. In a preferred embodiment at least three reference analytes are selected, including at least three reference volatile organic compounds such as cis-3-Hexen-1-ol, citronellol and Phenyl-Ethyl Alcohol (PEA).

At following step 204, these reference analytes are conditioned in reference fluid samples at concentrations exceeding a predefined level. The reference fluid samples are thus clustered accordingly.

Then, at step 206 several measurement cycles 100 are executed, for example at least thirty measurement cycles for each reference analyte in a preferred embodiment. According to a preferred embodiment too, at least ninety olfactory signatures are outputted, corresponding to the reference fluid samples, and stored in memory 40.

At following step 208, the stored olfactory signatures are annotated with their true respective reference analyte labels for clustering and scoring.

Then, at step 210 a validity index is computed for each of some or all of the reference fluid samples, i.e. for each of some or all of the corresponding stored olfactory signatures. It is advantageously based on intra-cluster and extra-cluster distances between olfactory signatures. As a consequence, the computing may be straightforward if there are few selected reactive sites at step 206, for example two or three. In that case raw or normalized olfactory signatures may be used. But if a greater number of reactive sites is selected, then it may be advisable to use simplified signatures resulting from a dimension reduction (principal component analysis, singular value decomposition, multidimensional scaling, or equivalent) to avoid computing complexity. Moreover, distances computed on simplified signatures resulting for example from a principal component analysis are generally more relevant.

For example, for each reference fluid sample $S_i$ a combination $a_i$ of intra-cluster distances computed between the corresponding olfactory signature and all other olfactory signatures of the same cluster is computed. Such a combination may be an average. For each reference fluid sample $S_i$ a combination $b_{i,j}$ of inter-cluster distances computed between the corresponding olfactory signature and all olfactory signatures of another cluster is computed for each other cluster (wherein index j identifies said other cluster). Such a combination may be an average. Then the minimum value $b_i = Min(b_{i,j})$ is selected. The resulting validity index $CQS_i$ for each reference fluid sample $S_i$ may then be computed as follows:

$$CQS_i = \frac{b_i - a_i}{Max(a_i, b_i)},$$

such that $-1 \le CQS_i \le 1$.

In a variant, outliers based on unrealistic values for $a_i$ and $b_i$ can be deleted so that the resulting validity index $CQS_i$ is computed only for each of some of the reference fluid samples. It can be considered that validity

index $CQS_i$ also represents a clustering quality score for reference fluid sample $S_i$. It can be expressed as a percentage.

At following step 212, a global clustering quality score CQS can be computed as a metrics based on all the validity indexes $CQS_i$ computed at step 210, for example as an average of these validity indexes. It can be expressed as a percentage and represents the quality of how sensor device 10 can discriminate multiple volatile organic compounds. In a variant, intermediary clustering quality scores $CQS_j$ can be computed for all clusters $C_j$, each based on the validity indexes $CQS_i$ computed for the reference fluid samples $S_i$ that it includes, for example as an average of these validity indexes $CQS_i$, and the global clustering quality score CQS can then be computed based on the intermediary clustering quality scores $CQS_j$, for example as an average of these intermediary clustering quality scores $CQS_j$.

As an option, the proposed metrics may further comprise an index of repeatability RTY. To this end, steps 202 to 206 can be repeated for a plurality of measurement runs for sensor device 10, for example ten or more, preferably between twenty and thirty. Each run is distant in time of about a predefined interval, for example 24 hours. However, this predefined interval may depend on a number of factors, such as the stability of the reference samples conditioning, the time needed for sensor device 10 to return to a steady state after each measurement, the stability of the environment, etc.

When steps 202 to 206 are executed for computing and storing olfactory signatures which are either raw or simplified but obtained from a principal component analysis of raw signatures, computing the index of repeatability RTY advantageously includes a step 214 of separately computing intensities of the stored olfactory signatures for the plurality of measurement runs and normalized signatures thereof by their respective intensities for the plurality of measurement runs too.

Then, at step 216, the index of repeatability RTY may be computed as:

- an index of dispersion of the previously computed intensities, which may be a relative variance around an average intensity for each reference sample for the plurality of measurement runs, expressed as a percentage, and/or

- an index of dispersion of the previously computed normalized signatures, which may be a relative variance around an average normalized signature for each reference sample for the plurality of measurement runs, expressed as a percentage too.

Advantageously, the index of repeatability RTY may include a clustering quality score CQS-RTY computed over the plurality of measurement runs, which may be a global clustering quality score computed from all annotated stored signatures for the plurality of measurement runs. In that case steps 202 to 208 must be repeated for the plurality of measurement runs for sensor device 10 and then steps 210 and 212 are executed using all annotated stored signatures obtained from said repetition of steps 202 to 208.

It will be noted that several other embodiments can be envisaged for each index of dispersion. It can be a variance, a standard deviation, a variance normalized by an average, a standard deviation normalized by an average, etc.

More generally, the index of repeatability RTY may be computed as a composite of at least one of the aforementioned index of dispersion of the previously computed intensities, index of dispersion of the previously computed normalized signatures and clustering quality score CQS-RTY computed over the plurality of measurement runs.

As an option, the proposed metrics may further comprise an index of reproducibility RDY. To this end, steps 202 to 206 can be repeated for a plurality of sensor devices, for example five or more, preferably between twenty and thirty.

When steps 202 to 206 are executed for computing and storing olfactory signatures which are either raw or simplified but obtained from a principal component analysis of raw signatures, computing the index of reproducibility RDY advantageously includes a step 218 of separately computing intensities of the stored olfactory signatures for the plurality of sensor devices and normalized signatures thereof by their respective intensities for the plurality of sensor devices too.

Then, at step 220, the index of reproducibility RDY may be computed as:

- an index of dispersion of the previously computed intensities, which may be a relative variance around an average intensity for each reference sample for the plurality of sensor devices, expressed as a percentage, and/or

- an index of dispersion of the previously computed normalized signatures, which may be a relative variance around an average normalized signature for each reference sample for the plurality of sensor devices, expressed as a percentage too.

Advantageously the index of reproducibility RDY may include a clustering quality scores CQS-RDY computed over the plurality of sensor devices, which may be a global clustering quality score computed from all annotated stored signatures for the plurality of sensor devices. In that case steps 202 to 208 must be repeated for the plurality of sensor devices and then steps 210 and 212 are executed using all annotated stored signatures obtained from said repetition of steps 202 to 208.

As for the index of repeatability RTY, several other embodiments can be envisaged for each index of dispersion in the index of reproducibility RDY.

More generally, the index of reproducibility RDY may be computed as a composite of at least one of the aforementioned index of dispersion of the previously computed intensities, index of dispersion of the previously computed normalized signatures and clustering quality score CQS-RDY computed over the plurality of sensor devices.

When the proposed metrics comprises both the index of repeatability RTY and the index of reproducibility RDY, steps 202 to 206, or steps 202 to 208, can be repeated for a plurality of measurement runs, each run being distant in time of about a predefined interval, and, for each measurement run, for a plurality of sensor devices. The resulting index of repeatability RTY and reproducibility RDY may also be computed as a composite of at least one of:

- an index of dispersion of the previously computed intensities, which may be a relative variance around an average intensity for each reference sample for the plurality of measurement runs and the plurality of sensor devices,

- an index of dispersion of the previously computed normalized signatures, which may be a relative variance around an average normalized signature for each reference sample for the plurality of measurement runs and the plurality of sensor devices, and

- a global clustering quality score computed over the plurality of measurement runs and the plurality of sensor devices.

More generally, when computing both the index of repeatability RTY and the index of reproducibility RDY, several scores can be computed at each measurement run and for each sensor device. These scores can be represented in a two-dimension diagram or table, one dimension for the successive measurement runs and one dimension for the sensor devices. From this representation, various statistics can be computed in each dimension or both of them for extracting any repeatability and/or reproducibility index based on the intensities, normalized signatures and/or global clustering quality scores. In particular, several global clustering quality scores CQS-RTY for repeatability can be computed for several sensor devices and then combined into an averaged global clustering quality score <CQS-RTY> for repeatability. Accordingly, several global clustering quality scores CQS-RDY for reproducibility can be computed for several measurement runs and then combined into an averaged global clustering quality score <CQS-RDY> for reproducibility.

As an option, the proposed metrics may further comprise an index of classification performance, for example based on a confusion matrix of reference signatures of reference fluid samples. To this end, the method 200 for administering a qualification test comprises a step 222 which is equivalent to steps 202 and 204, wherein several reference analytes, each of which defines a cluster, are selected and conditioned in reference fluid samples at concentrations exceeding a predefined level.

At following step 224, a classifier is trained using the reference fluid samples by executing several measurement cycles 100, for example at least thirty measurement cycles for each reference analyte, or even fifty or more in a preferred embodiment. Respective reference olfactory signatures are outputted, corresponding to the reference fluid samples, and stored in a database of reference signatures, for example in memory 40.

At following step 226, the classifier is tested using test fluid samples which are annotated for scoring, each corresponding to one of the reference fluid samples, by executing several measurement cycles 100, for example at least thirty. Respective test olfactory signatures are outputted, corresponding to the test fluid samples, and classified by association to one of said reference signatures, i.e. to one of said reference fluid samples.

At following step 228, a confusion matrix CM is completed using the results of classification step 226. As well known, each column of the confusion matrix represents for example the instances in a predicted class (or cluster) while each row represents the instances in an actual class (or cluster). Therefore CM(i,j) represents the number of test samples annotated as belonging to class/cluster $C_i$ that have been classified in class/cluster $C_j$.

Several indexes can be extracted from said confusion matrix CM for scoring the classification as a composite index of classification performance:

- a first general index of accuracy, which can be expressed as a percentage, corresponding to a ratio between the sum of first diagonal coefficients and the sum of all coefficients,

- a second class-depending index of sensitivity, which can be expressed as a percentage, corresponding for class/cluster $C_i$ to a ratio between CM(i,i) and the sum of i-th row coefficients,

- a third class-depending index of specificity, which can be expressed as a percentage, corresponding for class/cluster $C_i$ to a ratio between the sum of all coefficients CM(k,l), where $k \neq i$ and $l \neq i$, and the sum of all coefficients CM(k,l), where $k \neq i$, and

- a fourth class-depending index of precision, which can be expressed as a percentage, corresponding for class/cluster $C_i$ to a ratio between CM(i,i) and the sum of i-th column coefficients,

These indexes can be outputted in a table together with the abovementioned intermediary clustering quality scores computed for all classes/clusters $C_i$.

As an option, the proposed metrics may further comprise a signal-to-noise ratio. This ratio may be measured on one or more of the components of sensor device 10, in particular on the transducer 20. To this end, the method 200 for administering a qualification test for example comprises a step 230 of applying a reproducible stimulus on the transducer. In a non-limitative preferred embodiment, this stimulus is a reproducible pressure drop of, for example, 100 mBar. The signal outputted by the transducer 20 is measured during the stimulus and before or after the stimulus.

At following step 232 the signal outputted by the transducer 20 before or after the stimulus is for example processed to extract a variance value N which is considered the noise variance on stable baseline. During said same step, the signal outputted by the transducer 20 during the stimulus is for example processed to extract an average value S which is considered the average power during stimulus. The signal-to-noise ratio can then be computed and expressed in dB as SNR = 10.log(S/N).

It is therefore possible to compare the SNR of sensor devices from different technologies.

As an option, the proposed metrics may further comprise a limit of detection index. Such an index is composite, each component specific to a given analyte. The limit of detection of an analyte is the lowest concentration of this analyte, for example in a set of predefined concentrations, that sensor device 10 can distinguish from its absence in a sample. To this end, the method 200 for administering a qualification test comprises a step 234 wherein several reference analytes are selected and conditioned in reference fluid samples at different concentrations or dilutions. In a preferred embodiment at least three reference analytes are selected, including at least three reference volatile organic compounds such as cis-3-Hexen-1-ol, citronellol and PEA. Depending on which dilution strategy is employed, the unit may be either ppmV (volumic parts-per-million) or percentage of liquid dilution (mass ratio between solvent mass and compound mass). Liquid dilution will be preferred for flavors and fragrance applications, while ppmV will be mainly used in environmental applications. If liquid dilution is chosen, successive dilutions with decade ratios ($1, 10^{-1}, 10^{-2}, 10^{-3}, ..., 0$) of each analyte in a specific neutral solvent may be prepared as fluid samples.

Then at step 236 several measurement cycles 100 are executed, for example at least seventy measurement cycles for each dilution fluid sample of each analyte in a preferred embodiment. Measurement cycles include estimation of a detection index which is advantageously a scalar value that statistically increases with the increasing presence of analyte in the dilution sample. Its definition and algorithmic estimation is known from those skilled in the art, highly dependent on the technology of sensor device 10, further dependent on the application, so that it will not be detailed. Each set of measurement cycles executed for each analyte dilution fluid sample outputs a distribution of values in the detection index scale.

At following step 238 a threshold T for decision is determined within the determination index scale for each analyte. Given the outputted distribution of the dilution fluid sample with no analyte, T is the detection index value under which is a predefined percentage, for example 95%, of said outputted distribution. It means that this threshold T is defined to obtain a false alarm probability of 5% for the dilution fluid sample with no analyte.

At following step 240, the limit of detection LOD is determined for each analyte as the lowest dilution fluid sample of said analyte the outputted distribution of which is more than a predefined percentage, for example 95%, above threshold T. It means that LOD is the lowest dilution sample of said analyte which outputs a correct detection probability of more than 95%.

A method 300 for manufacturing sensor device 10 will now be detailed with reference to Fig. 7.

At a first step 302, sensor device 10 is provided but not certified. It is configured to input a fluid sample and to output a corresponding signature obtained from an electric signal characterizing the fluid sample. It further comprises at least one reactive site, preferably several, and at least one transducer configured to measure at least one physical property change induced by an interaction of any fluid sample with said at least one reactive site and to output said electric signal.

Then at step 304, the method 200 for administering a qualification test is applied to sensor device 10. The metrics computed by administering the qualification test at least includes the global clustering quality score CQS. It may further include at least one of other indexes RTY, RDY, CM, SNR and LOD. All theses scores and indexes may further be combined into one global score, for instance a scalar combination thereof. In that case, it may be advantageous to previously express all scores and indexes homogeneously, for example as percentages.

At step 306, the computed metrics is compared to at least one predefined qualifying criterion, which may be one or more thresholds for simple cases.

Finally, sensor device 10 is certified at step 308 as far as the computed metrics matches said at least one predefined qualifying criterion, for example as far as the computed metrics matches or exceeds a predefined threshold when said threshold is a floor, or matches or keeps under a predefined threshold when said threshold is a ceiling.

It clearly appears that a sensor device and a method for administering a qualification test to said sensor device such as those described hereinabove make it possible to impartially compare different technologies or manufacturing processes, or guarantee a certain objective level of qualify, in a technical field wherein qualifying sensor devices is usually considered highly subjective.

## Claims

1. A method (200) for administering a qualification test to at least one sensor (10), the at least one sensor configured to input a fluid sample and to output a corresponding signature obtained from an electric signal (S) characterizing the fluid sample, the sensor (10) comprising:

   - at least one reactive site (18);
   - at least one transducer (20) configured to measure at least one physical property change induced by an interaction of the fluid sample with said at least one reactive site (18) and to output said electric signal (S);

   wherein the method (200) comprises:

   - inputting a plurality of reference fluid samples, which are each *a priori* clustered in one of several predefined clusters, for obtaining, from the at least one sensor (10), a plurality of respective signatures which are then also *a priori* clustered accordingly,
   - computing a metrics that comprises a clustering quality score (CQS) of said plurality of a priori clustered respective signatures of the plurality of reference fluid samples, wherein said clustering quality score (CQS) is based on intra-cluster and extra-cluster distances between said *a priori* clustered respective signatures.

2. The method (200) for administering a qualification test as claimed in claim 1, wherein the clustering quality score (CQS) is computed based on a validity index combining an intra-cluster distance and an extra-cluster distance computed for each one of some or all of the *a priori* clustered respective signatures of the plurality of reference fluid samples.

3. The method (200) for administering a qualification test as claimed in claim 1 or 2, wherein the metrics further comprises one or more of a signal-to-noise ratio (SNR) measured on one or more components of the at least one sensor (10), a limit of detection (LOD) of several reference analytes by the at least one sensor (10), an index of repeatability (RTY) of a plurality of measurement runs on a single one of the at least one sensor (10) and an index of reproducibility (RDY) of a plurality of measurement runs performed on a plurality of sensors (10).

4. The method (200) for administering a qualification test as claimed in any one of claims 1 to 3, further comprising inputting the signature of the fluid sample to a classifier connected to a database of reference signatures of respective reference fluid samples in order to have the signature of the fluid sample associated to one of said reference signatures, wherein the metrics further comprises an index of classification performance (CM).

5. The method (200) for administering a qualification test as claimed in claim 4, wherein the index of classification performance (CM) is based on a confusion matrix of the reference signatures.

6. The method (200) for administering a qualification test as claimed in any one of claims 1 to 5, wherein the plurality of reference fluid samples each comprise a volatile organic compound.

7. The method (200) for administering a qualification test as claimed in any one of claims 1 to 6, wherein computing the metrics comprises:

   a) selecting (202) a plurality of reference analytes,
   b) conditioning (204) them in the plurality of reference fluid samples, with the plurality of reference analytes in concentrations exceeding a predefined level,
   c) outputting (206) the corresponding signatures of the plurality of reference fluid samples over a plurality of measurement cycles,
   d) annotating (208) the corresponding signatures of the plurality of reference fluid samples with reference analyte labels for *a priori* clustering,
   e) computing (210), for each reference fluid sample $S_i$, a combination $a_i$ of intra-cluster distances for the corresponding signature within the cluster thereof,
   f) computing (210), for each reference fluid sample $S_i$, at least one combination of extra-cluster distances $b_{i,j}$ for the corresponding signature with the signatures in the cluster(s) different from the cluster thereof,
   g) computing (210) a validity index combining the intra-cluster distances and the extra-cluster distances for each of some or all of the reference fluid samples.

8. The method (200) for administering a qualification test as claimed in claim 7, wherein the clustering quality score (CQS) is based (212) on the one or more validity index(es) computed at step g).

9. The method (200) for administering a qualification test as claimed in claim 7 or 8, further comprising repeating (202, 204, 206) at least steps a) through c), for example steps a) to d), for a plurality of measurement runs for a same sensor, each run being distant in time of about a predefined interval, wherein the metrics further comprises an index of repeatability (RTY) computed as a composite of at least one of:

- a clustering quality score computed over the plurality of measurement runs;
- an index of dispersion of intensities of the signatures of the fluid samples for the plurality of measurement runs; and
- an index of dispersion of the signatures normalized by their respective intensities of the fluid samples for the plurality of measurement runs.

10. The method (200) for administering a qualification test as claimed in claim 7 or 8, further comprising repeating (202, 204, 206) at least steps a) through c), for example steps a) to d), for a plurality of sensors, wherein the metrics further comprises an index of reproducibility (RDY) computed as a composite of at least one of:

- a clustering quality score computed over the plurality of sensors;
- an index of dispersion of intensities of the signatures of the fluid samples for the plurality of sensors; and
- an index of dispersion of the signatures normalized by their respective intensities of the fluid samples for the plurality of sensors.

11. The method (200) for administering a qualification test as claimed in claims 9 and 10, further comprising repeating (202, 204, 206) at least steps a) through c), for example steps a) to d), for a plurality of measurement runs, each run being distant in time of about a predefined interval, and, for each measurement run, repeating (202, 204, 206) steps a) through c), for example steps a) to d), for a plurality of sensors, wherein the metrics further comprises an index of repeatability and reproducibility (RTY, RDY) computed as a composite of at least one of:

- a clustering quality score computed over the plurality of measurement runs and the plurality of sensors;
- an index of dispersion of intensities of the signatures of the fluid samples for the plurality of measurement runs and the plurality of sensors; and
- an index of dispersion of the signatures normalized by their respective intensities of the fluid samples for the plurality of measurement runs and the plurality of sensors.

12. A method (300) for manufacturing a certified sensor (10), comprising:

- providing (302) a sensor (10) configured to input a fluid sample and to output a corresponding signature obtained from an electric signal (S) characterizing the fluid sample, the sensor (10) comprising at least one reactive site (18) and at least one transducer (20) configured to measure at least one physical property change induced by an interaction of the fluid sample with said at least one reactive site and to output said electric signal (S);
- applying (304) the method (200) for administering a qualification test as claimed in any one of claims 1 to 11 to the sensor (10);
- checking (306) that the metrics computed by administering (200) the qualification test matches at least one predefined qualifying criterion; and
- certifying (308) the sensor (10) as far as said metrics matches said at least one predefined qualifying criterion.

13. The method (300) for manufacturing a certified sensor (10) as claimed in claim 12, comprising providing (302) the sensor (10) with a plurality of reactive sites (18) and wherein each of said reactive sites comprises a chemical component that has adsorbing properties for a volatile organic compound, such as a peptide immobilized on a substrate, or a polymer coated on a surface.

14. The method (300) for manufacturing a certified sensor (10) as claimed in claim 12 or 13, comprising providing (302) the sensor (10) with the at least one transducer (20) comprising:

- a surface plasmon resonance imaging system configured to measure a change in a refractive index thanks to a plasmonic effect; or
- a Mach-Zehnder interferometer configured to measure a phase shift due to a change in a refractive index; or
- a nano- or micro-electromechanical system configured to measure a change in resonance frequency of a vibrating membrane thereof.

**Patentansprüche**

1. Verfahren (200) zum Abwickeln eines Qualifikationstests an mindestens einen Sensor (10), wobei der mindestens eine Sensor konfiguriert ist, um eine Fluidprobe einzugeben und eine entsprechende Signatur auszugeben, die von einem elektrischen Signal (S) erhalten wird, welches die Fluidprobe charakterisiert, wobei der Sensor (10) umfasst:

- mindestens eine reaktive Stelle (18);
- mindestens einen Wandler (20), der konfiguriert ist, um mindestens eine Änderung einer physikalischen Eigenschaft zu messen, die durch eine Interaktion der Fluidprobe mit der mindestens einen reaktiven Stelle (18) ausgelöst wird, und um das elektrische Signal (S) auszugeben;

wobei das Verfahren (200) umfasst:

- Eingeben eine Vielzahl von Referenzfluidproben, die jeweils a priori in einem von verschiedenen vordefinierten Clustern geclustert sind, zum Erhalten von dem mindestens einen Sensor (10) einer Vielzahl von jeweiligen Signaturen, die danach auch dementsprechend a priori geclustert werden,
- Berechnen einer Metrik, welche eine Clusterbildungs-Qualitätskennzahl (CQS) der Vielzahl von a priori geclusterten jeweiligen Signaturen der Vielzahl von Referenzfluidproben umfasst, wobei die Clusterbildungs-Qualitätskennzahl (CQS) auf clusterinternen und clusterexternen Abständen zwischen den jeweiligen a priori geclusterten Signaturen basiert.

2. Verfahren (200) zum Abwickeln eines Qualifikationstests nach Anspruch 1, wobei die Clusterbildungs-Qualitätskennzahl (CQS) basierend auf einem Gültigkeitsindex berechnet wird, der einen clusterinternen Abstand und einen clusterexternen Abstand kombiniert, die für jede von einigen oder allen der jeweiligen a priori geclusterten Signaturen der Vielzahl von Referenzfluidproben berechnet werden.

3. Verfahren (200) zum Abwickeln eines Qualifikationstests nach Anspruch 1 oder 2, wobei die Metrik weiter eines oder mehr von einem Signal-Geräusch-Verhältnis (SNR), das an einer oder mehreren Komponenten des mindestens einen Sensors (10) gemessen wird, einer Detektionsgrenze (LOD) verschiedener Referenz-Analyten durch den mindestens einen Sensor (10), einem Wiederholbarkeitsindex (RTY) einer Vielzahl von Messläufen auf einem einzigen des mindestens einen Sensors (10), und einem Reproduzierbarkeitsindex (RDY) einer Vielzahl von Messläufen, die auf einer Vielzahl von Sensoren (10) durchgeführt werden, umfasst.

4. Verfahren (200) zum Abwickeln eines Qualifikationstests nach einem der Ansprüche 1 bis 3, weiter umfassend das Eingeben der Signatur der Fluidprobe in einen Klassifikator, der mit einer Datenbank von Referenzsignaturen jeweiliger Referenzfluidproben verbunden ist, um die Signatur der Fluidprobe aufzuweisen, die einer der Referenzsignaturen zugeordnet ist, wobei die Metrik weiter einen Index für eine Klassifizierungsleistung (CM) umfasst.

5. Verfahren (200) zum Abwickeln eines Qualifikationstests nach Anspruch 4, wobei der Index für eine Klassifizierungsleistung (CM) auf einer Konfusionsmatrix der Referenzsignaturen basiert.

6. Verfahren (200) zum Abwickeln eines Qualifikationstests nach einem der Ansprüche 1 bis 5, wobei die Vielzahl von Referenzfluidproben jeweils eine flüchtige organische Verbindung umfassen.

7. Verfahren (200) zum Abwickeln eines Qualifikationstests nach einem der Ansprüche 1 bis 6, wobei das Berechnen der Metrik umfasst:

a) Auswählen (202) einer Vielzahl von Referenz-Analyten,
b) deren Konditionieren (204) in der Vielzahl von Referenzfluidproben, wobei die Vielzahl von Referenz-Analyten in der Konzentration ein vordefiniertes Niveau übersteigen,
c) Ausgeben (206) der entsprechenden Signaturen der Vielzahl von Referenzfluidproben über eine Vielzahl von Messzyklen,
d) Versehen (208) der entsprechenden Signaturen der Vielzahl von Referenzfluidproben mit Referenz-Analyten-Kennzeichnungen für a priori Clustern.
e) Berechnen (210) für jede Referenzfluidprobe $S_i$ einer Kombination $a_i$ von clusterinternen Abständen für die entsprechende Signatur innerhalb des Clusters davon,
f) Berechnen (210) für jede Referenzfluidprobe $S_i$ mindestens einer Kombination von clusterexternen Abständen $b_{i,j}$ für die entsprechende Signatur mit den Signaturen im (in den) Cluster(n), (der) die sich von dem Cluster davon unterscheidet (en),
g) Berechnen (210) eines Gültigkeitsindex, welcher die clusterinternen Abstände und die clusterexternen Abstände für jede von einigen oder allen der Referenzfluidproben kombiniert.

8. Verfahren (200) zum Abwickeln eines Qualifikationstests nach Anspruch 7, wobei die Clusterbildungs-Qualitätskennzahl (CQS) auf dem einen oder mehreren in Schritt g) berechneten Gültigkeitsindex (-indizes) basiert (212).

9. Verfahren (200) zum Abwickeln eines Qualifikationstests nach Anspruch 7 oder 8, weiter umfassend das Wiederholen (202, 204, 206) von mindestens der Schritte a) bis c), beispielsweise der Schritte a) bis d) für eine Vielzahl von Messläufen für einen gleichen Sensor, wobei jeder Lauf zeitlich um etwa ein vordefiniertes Intervall beabstandet ist, wobei die Metrik weiter einen Wiederholbarkeitsindex (RTY) umfasst, der als Verbund von mindestens einem berechnet wird, von:

- einer Clusterbildungs-Qualitätskennzahl, die über die Vielzahl von Messläufen berechnet wird;

- einem Index für die Streuung von Intensitäten der Signaturen der Fluidproben für die Vielzahl von Messläufen; und
- einem Index für die Streuung der Signaturen, die durch ihre jeweiligen Intensitäten der Fluidproben für die Vielzahl von Messläufen normalisiert sind.

10. Verfahren (200) zum Abwickeln eines Qualifikationstests nach Anspruch 7 oder 8, weiter umfassend das Wiederholen (202, 204, 206) mindestens der Schritte a) bis c), beispielsweise der Schritte a) bis d) für eine Vielzahl von Sensoren, wobei die Metrik weiter einen Reproduzierbarkeitsindex (RDY) umfasst, der als Verbund von mindestens einem berechnet wird, von:

- einer Clusterbildungs-Qualitätskennzahl, die über die Vielzahl von Sensoren berechnet wird;
- einem Index für die Streuung von Intensitäten der Signaturen der Fluidproben für die Vielzahl von Sensoren; und
- einem Index für die Streuung der Signaturen, die durch ihre jeweiligen Intensitäten der Fluidproben für die Vielzahl von Sensoren normalisiert sind.

11. Verfahren (200) zum Abwickeln eines Qualifikationstests nach den Ansprüchen 9 und 10, weiter umfassend das Wiederholen (202, 204, 206) mindestens der Schritte a) bis c), beispielsweise der Schritte a) bis d) für eine Vielzahl von Messläufen, wobei jeder Lauf zeitlich um etwa ein vordefiniertes Intervall beabstandet ist, und für jeden Messlauf das Wiederholen (202, 204, 206) der Schritte a) bis c), beispielsweise der Schritte a) bis d) für eine Vielzahl von Sensoren, wobei die Metrik weiter einen Wiederholbarkeits- und Reproduzierbarkeitsindex (RTY, RDY) umfasst, die als Verbund von mindestens einem berechnet werden, von:

- einer Clusterbildungs-Qualitätskennzahl, die über die Vielzahl von Messläufen und die Vielzahl von Sensoren berechnet wird;
- einem Index für die Streuung von Intensitäten der Signaturen der Fluidproben für die Vielzahl von Messläufen und die Vielzahl von Sensoren; und
- einem Index für die Streuung der Signaturen, die durch ihre jeweiligen Intensitäten der Fluidproben für die Vielzahl von Messläufen und die Vielzahl von Sensoren normalisiert sind.

12. Verfahren (300) zum Herstellen eines zertifizierten Sensors (10), umfassend:

- Bereitstellen (302) eines Sensors (10), der konfiguriert ist, um eine Fluidprobe einzugeben und eine entsprechende Signatur auszugeben, die von einem elektrischen Signal (S) erhalten wird, welches die Fluidprobe charakterisiert, wobei der Sensor (10) mindestens eine reaktive Stelle (18) und mindestens einen Wandler (20) umfasst, der konfiguriert ist, um mindestens eine Änderung einer physikalischen Eigenschaft zu messen, die durch eine Interaktion der Fluidprobe mit der mindestens einen reaktiven Stelle ausgelöst wird, und um das elektrische Signal (S) auszugeben;
- Anwenden (304) des Verfahrens (200) zum Abwickeln eines Qualifikationstests nach einem der Ansprüche 1 bis 11 auf den Sensor (10);
- Überprüfen (306), dass die Metrik, die durch Abwickeln (200) des Qualifikationstests berechnet wird, das mindestens eine vordefinierte Qualifikationskriterium erfüllt; und
- Zertifizieren (308) des Sensors (10) sofern die Metrik das mindestens eine vordefinierte Qualifikationskriterium erfüllt.

13. Verfahren (300) zum Herstellen eines zertifizierten Sensors (10) nach Anspruch 12, umfassend das Bereitstellen (302) des Sensors (10) mit einer Vielzahl von reaktiven Stellen (18) und wobei jede der reaktiven Stellen eine chemische Komponente umfasst, die absorbierende Eigenschaften für eine flüchtige organische Verbindung, wie ein auf einem Substrat immobilisiertes Peptid, oder ein auf einer Oberfläche beschichtetes Polymer aufweist.

14. Verfahren (300) zum Herstellen eines zertifizierten Sensors (10) nach Anspruch 12 oder 13, umfassend das Bereitstellen (302) des Sensors (10) mit dem mindestens einen Wandler (20), umfassend:

- ein Oberflächenplasmaresonanz-Bildgebungssystem, das konfiguriert ist, um dank eines plasmonischen Effekts eine Änderung eines Brechungsindex zu messen; oder
- einen Mach-Zehnder-Interferometer, der konfiguriert ist, um eine Phasenverschiebung aufgrund einer Änderung eines Brechungsindex zu messen; oder
- ein nano- oder mikroelektromechanisches System, das konfiguriert ist, um eine Änderung der Resonanzfrequenz einer Schwingungsmembran davon zu messen.

**Revendications**

1. Procédé (200) d'administration d'un test de qualification à au moins un capteur (10), ledit au moins un capteur étant configuré pour recevoir un échantillon de fluide et pour fournir une signature correspondante obtenue à partir d'un signal électrique (S) caractérisant l'échantillon de fluide, le capteur (10) comprenant :

- au moins un site réactif (18) ;
- au moins un transducteur (20) configuré pour mesurer au moins un changement de propriété physique induit par une interaction de l'échantillon de fluide avec ledit au moins un site réactif (18) et pour émettre ledit signal électrique (S) ;

le procédé (200) comprenant :

- la réception d'une pluralité d'échantillons de fluides de référence, qui sont chacun classés a *priori* dans l'un de plusieurs groupes prédéfinis, pour obtenir, à partir dudit au moins un capteur (10), une pluralité de signatures respectives qui sont alors également regroupées a *priori* en conséquence,
- le calcul d'une métrique comprenant un score de qualité de regroupement (CQS) de ladite pluralité de signatures respectives regroupées a *priori* de la pluralité d'échantillons de fluides de référence, ledit score de qualité de regroupement (CQS) étant basé sur des distances intra-cluster et extra-cluster entre lesdites signatures respectives regroupées a *priori.*

2. Procédé (200) d'administration d'un test de qualification selon la revendication 1, dans lequel le score de qualité de regroupement (CQS) est calculé sur la base d'un indice de validité combinant une distance intra-cluster et une distance extra-cluster calculée pour chacune d'une partie ou de la totalité des signatures respectives regroupées a *priori* de la pluralité d'échantillons de fluides de référence.

3. Procédé (200) d'administration d'un test de qualification selon la revendication 1 ou 2, dans lequel la métrique comprend en outre l'un ou plusieurs d'un rapport signal sur bruit (SNR) mesuré sur un ou plusieurs composants dudit au moins un capteur (10), d'une limite de détection (LOD) de plusieurs analytes de référence par ledit au moins un capteur (10), d'un indice de répétabilité (RTY) d'une pluralité de mesures effectuées sur un seul dudit au moins un capteur (10) et d'un indice de reproductibilité (RDY) d'une pluralité de mesures effectuées sur une pluralité de capteurs (10).

4. Procédé (200) d'administration d'un test de qualification selon l'une quelconque des revendications 1 à 3, comprenant en outre la fourniture de la signature de l'échantillon de fluide à un classificateur connecté à une base de données de signatures de référence d'échantillons de fluides de référence respectifs afin d'associer la signature de l'échantillon de fluide à l'une desdites signatures de référence, la métrique comprenant en outre un indice de performance du regroupement (CM).

5. Procédé (200) d'administration d'un test de qualification selon la revendication 4, dans lequel l'indice de performance du regroupement (CM) est basé sur une matrice de confusion des signatures de référence.

6. Procédé (200) d'administration d'un test de qualification selon l'une quelconque des revendications 1 à 5, dans lequel les échantillons de fluides de référence comprennent chacun un composé organique volatil.

7. Procédé (200) d'administration d'un test de qualification selon l'une quelconque des revendications 1 à 6, dans lequel le calcul de la métrique comprend :

a) la sélection (202) d'une pluralité d'analytes de référence,
b) leur conditionnement (204) dans la pluralité d'échantillons de fluides de référence, avec la pluralité d'analytes de référence dans des concentrations supérieures à un niveau prédéfini,
c) la fourniture (206) des signatures correspondantes de la pluralité d'échantillons de fluides de référence sur une pluralité de cycles de mesure,
d) l'annotation (208) des signatures correspondantes de la pluralité d'échantillons de fluides de référence avec des étiquettes d'analytes de référence pour un regroupement a *priori,*
e) le calcul (210), pour chaque échantillon de fluide de référence $S_i$, d'une combinaison $a_i$ de distances intra-cluster pour la signature correspondante au sein de son groupe,
f) le calcul (210), pour chaque échantillon de fluide de référence $S_i$, d'au moins une combinaison de distances extra-cluster $b_{i,j}$ pour la signature correspondante avec les signatures du (des) groupe(s) différent(s) de son groupe,
g) le calcul (210) d'un indice de validité combinant les distances intra-cluster et les distances extra-cluster pour chacune d'une partie ou de la totalité des échantillons de fluides de référence.

8. Procédé (200) d'administration d'un test de qualification selon la revendication 7, dans lequel le score de qualité de regroupement (CQS) est basé (212) sur le ou les indice(s) de validité calculé(s) à l'étape g).

9. Procédé (200) d'administration d'un test de qualification selon la revendication 7 ou 8, comprenant en outre la répétition (202, 204, 206) d'au moins les étapes a) à c), par exemple les étapes a) à d), pour plusieurs séries de mesures pour un même capteur, chaque série étant distante dans le temps d'environ un intervalle prédéfini, la métrique comprenant en outre un indice de répétabilité (RTY) calculé comme un composite d'au moins l'un des éléments suivants :

- un score de qualité de regroupement calculé sur la pluralité des séries de mesures ;
- un indice de dispersion d'intensités des signatures des échantillons de fluides pour la pluralité de séries de mesures ; et
- un indice de dispersion des signatures normalisées par leurs intensités respectives des échantillons de fluides pour la pluralité de séries de mesures.

10. Procédé (200) d'administration d'un test de qualification selon la revendication 7 ou 8, comprenant en outre la répétition (202, 204, 206) d'au moins les étapes a) à c), par exemple les étapes a) à d), pour une pluralité de capteurs, la métrique comprenant en outre un indice de reproductibilité (RDY) calculé comme un composite d'au moins l'un des éléments suivants :

- un score de qualité de regroupement calculé sur la pluralité de capteurs ;
- un indice de dispersion d'intensités des signatures des échantillons de fluides pour la pluralité de capteurs ; et
- un indice de dispersion des signatures normalisées par leurs intensités respectives des échantillons de fluides pour la pluralité de capteurs.

11. Procédé (200) d'administration d'un test de qualification selon les revendications 9 et 10, comprenant en outre la répétition (202, 204, 206) d'au moins les étapes a) à c), par exemple les étapes a) à d), pour plusieurs séries de mesures, chaque série étant distante dans le temps d'environ un intervalle prédéfini, et, pour chaque série de mesures, en répétant (202, 204, 206) les étapes a) à c), par exemple les étapes a) à d), pour une pluralité de capteurs, la métrique comprenant en outre un indice de répétabilité et de reproductibilité (RTY, RDY) calculé comme un composite d'au moins l'un des éléments suivants :

- un score de qualité de regroupement calculé sur la pluralité de séries de mesures et la pluralité de capteurs ;
- un indice de dispersion d'intensités des signatures des échantillons de fluides pour la pluralité de séries de mesures et la pluralité de capteurs ; et
- un indice de dispersion des signatures normalisées par leurs intensités respectives des échantillons de fluides pour la pluralité de séries de mesures et la pluralité de capteurs.

12. Procédé (300) de fabrication d'un capteur certifié (10), comprenant :

- la fourniture (302) d'un capteur (10) configuré pour recevoir un échantillon de fluide et fournir une signature correspondante obtenue à partir d'un signal électrique (S) caractérisant l'échantillon de fluide, le capteur (10) comprenant au moins un site réactif (18) et au moins un transducteur (20) configuré pour mesurer au moins un changement de propriété physique induit par une interaction de l'échantillon de fluide avec ledit au moins un site réactif et pour fournir ledit signal électrique (S) ;
- l'application (304) au capteur (10) du procédé (200) d'administration d'un test de qualification selon l'une quelconque des revendications 1 à 11 ;
- la vérification (306) que la métrique calculée par l'administration (200) du test de qualification est à la hauteur d'au moins un critère de qualification prédéfini ; et
- la certification (308) du capteur (10) dans la mesure où ladite métrique est à la hauteur dudit au moins un critère de qualification prédéfini.

13. Procédé (300) de fabrication d'un capteur certifié (10) selon la revendication 12, comprenant la fourniture (302) du capteur (10) avec une pluralité de sites réactifs (18) et dans lequel chacun de ces sites réactifs comprend un composant chimique qui a des propriétés d'adsorption d'un composé organique volatil, tel qu'un peptide immobilisé sur un substrat, ou un polymère enduit sur une surface.

14. Procédé (300) de fabrication d'un capteur certifié (10) selon la revendication 12 ou 13, comprenant la fourniture (302) du capteur (10) avec au moins un transducteur (20) comprenant :

- un système d'imagerie par résonance plasmonique de surface configuré pour mesurer un changement d'indice de réfraction grâce à un effet plasmonique ; ou
- un interféromètre de Mach-Zehnder configuré pour mesurer un déphasage dû à un changement d'indice de réfraction ; ou
- un système nano- ou micro-électromécanique configuré pour mesurer un changement de fréquence de résonance d'une membrane vibrante de ce système.

## Figure 1

## Figure 2

## Figure 3

## Figure 4

## Figure 5

## Figure 6

200

| 202 |
| 204 |
| 206 |

40

| 208 |

| 214 | | 218 | | 210 | | 222 |
| 216 | | 220 | | 212 | | 224 |

| | | | | 226 | | 230 | | 234 |
| | | | | 228 | | 232 | | 236 |
| | | | | | | | | 238 |
| | | | | | | | | 240 |

RTY        RDY        CQS        CM        SNR        LOD

## Figure 7

300

| 302 |
| 304 |
| 306 |
| 308 |

**EP 3 968 018 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019053366 A1